# EUROPEAN PATENT APPLICATION

(11) **EP 4 283 568 A1**
(43) Date of publication of application: **29.11.2023**
(21) Application number: 22742074.2
(22) Date of filing: 13.01.2022
(51) Int. Cl.: G06T 19/20

(54) **METHOD AND DEVICE FOR ADJUSTING DENTAL MODEL**

(30) Priority: 20.01.2021 CN 202110077663
(71) Applicant: Shining 3D Tech Co., Ltd., Hangzhou, Zhejiang 311258 (CN)
(72) Inventor: CHEN, Xiaojun, Hangzhou, Zhejiang 311258 (CN); SHAO, Maozhen, Hangzhou, Zhejiang 311258 (CN); JIA, Yanming, Hangzhou, Zhejiang 311258 (CN)
(74) Representative: Grünecker Patent- und Rechtsanwälte PartG mbB
(86) International application number: PCT/CN2022/071878
(87) International publication number: WO 2022/156595

(57) **Abstract**

Provided is a method and device for adjusting a dental model. The method includes: acquiring an initial edge line of a dental model to be adjusted (S102); and adjusting at least part of the initial edge line to obtain an adjusted edge line (S104).

## Description

### Cross Reference

The present disclosure claims the priority of the patent application No. 202110077663.3, filed with China National Intellectual Property Administration on January 20, 2021, and entitled "Method and Device for Adjusting Dental Model", which is incorporated herein by reference in its entirety.

### Technical Field

The present disclosure relates to the field of model design, and specifically relates to a method and device for adjusting a dental model.

### Background

In the process of clinical oral diagnosis, treatment and restoration, an oral impression is an important information storage source, and an optical impression is one of the oral impressions. At present, the optical impression may be obtained by an intraoral scanner or a desktop scanner. However, the optical impressions obtained by the existing methods have the problem of insufficient exposure of an edge line of a dental model, so it is necessary to trim the dental model.

In the related art, the dental model may be trimmed by means of a toolkit for mesh deformation in dental CAD software. This toolkit allows the user to deform data in a certain area according to their own experience, for example, adjust a part of the model to be concave or convex. Moreover, the user may also set the intensity and range of deformation, and finally make the edge line of the dental model according to the experience.

As can be seen from the above, this solution requires an operator to have the experience of model trimming and to estimate values to be corrected of the dental model according to the experience. Moreover, compared with the method of trimming a real plaster model, trimming a dental model in a computer by deformation lacks intuitive feedback, so it is impossible to evaluate whether the dental model has been trimmed properly. In addition, the degree of trimming to the dental model depends entirely on the evaluation of the operator, the process of trimming the dental model is greatly affected by human factors, and there is an uncertainty, so that the dental model cannot be trimmed accurately.

For the above problems, no effective solutions have been proposed yet.

### Summary

One embodiment of the present disclosure provides a method and device for adjusting a dental model to at least solve the technical problem of inaccurate edge line after trimming the dental model in the prior art.

One embodiment of the present disclosure provides a method for adjusting a dental model, including: acquiring an initial edge line of a dental model to be adjusted; and adjusting the dental model to be adjusted based on the initial edge line to obtain a target model; and adjusting at least part of the initial edge line based on the target model to obtain an adjusted edge line.

Alternatively, the method for adjusting a dental model further includes: projecting the dental model to be adjusted to obtain a target area corresponding to the dental model to be adjusted, the target area at least including a deformation area; and determining the initial edge line according to the target area.

Alternatively, the method for adjusting a dental model further includes: detecting whether the dental model to be adjusted has a defect; adjusting, in a case that the dental model to be adjusted does not have the defect, the dental model to be adjusted based on the initial edge line to obtain the adjusted edge line; and performing, in a case that the dental model to be adjusted has the defect, a filling operation on the dental model to be adjusted based on the defect to obtain a filled dental model to be adjusted, adjusting the filled dental model to be adjusted based on the initial edge line to obtain a target model, and adjusting the at least part of the initial edge line based on the target model to obtain the adjusted edge line.

Alternatively, the method for adjusting a dental model further includes: acquiring a target area of the dental model to be adjusted and a depth map corresponding the dental model to be adjusted; determining, according to the depth map, whether there is an occlusion area in the target area of the dental model to be adjusted; and determining, in a case that there is the occlusion area in the target area of the dental model to be adjusted, that the dental model to be adjusted has the defect.

Alternatively, the method for adjusting a dental model further includes: performing, in a case that the dental model to be adjusted has the defect, a culling operation on noise information in a defect area to obtain a first defect area, the defect area including a plurality of occlusion points; mixing isolated occlusion points in the first defect area to obtain a second defect area; and performing a filling operation on the second defect area to obtain the filled dental model to be adjusted.

Alternatively, the method for adjusting a dental model further includes: determining, in a case that the dental model to be adjusted does not have the defect and an accuracy of a whole of the initial edge line is lower than a preset accuracy, that a movement mode of the at least part of the initial edge line is a first movement mode, the first movement mode being used for controlling the whole of the initial edge line to a preset position.

Alternatively, the method for adjusting a dental model further includes: determining, in a case that the dental model to be adjusted does not have the defect and an accuracy of the part of the initial edge line is lower than a preset accuracy, that a movement mode of the at least part of the initial edge line is a second movement mode.

Alternatively, the method for adjusting a dental model further includes: determining, based on the target model, a start position and an end position of the at least part of the initial edge line; determining a first edge line according to the start position and the end position, an accuracy of the first edge line being lower than the preset accuracy; and controlling the first edge line to move to a preset position to obtain the adjusted edge line.

Alternatively, the method for adjusting a dental model further includes: determining, in a case that the dental model to be adjusted has the defect and an accuracy of the part of the initial edge line is lower than a preset accuracy, that a movement mode of the at least part of the initial edge line is a third movement mode.

Alternatively, the method for adjusting a dental model further includes: determining, based on the target model, a start position and an end position of the at least part of the initial edge line; determining a second edge line according to the start position and the end position, an accuracy of the second edge line being lower than the preset accuracy; determining critical control points from a plurality of control points contained in the second edge line; determining a preset position corresponding to each of the critical control points; and controlling each of the critical control points to move to the preset position corresponding to each of the critical control points to obtain the adjusted edge line.

Alternatively, the method for adjusting a dental model further includes: determining target positions corresponding to non-critical control points contained in the second edge line; and determining that the non-critical control points are moved to the target positions to obtain the adjusted edge line.

One embodiment of the present disclosure further provides an device for adjusting a dental model, including: an acquisition module, configured to acquire an initial edge line of a dental model to be adjusted; and an adjustment module, configured to adjust at least part of the initial edge line to obtain an adjusted edge line.

One embodiment of the present disclosure further provides a non-volatile storage medium, storing a computer program therein. The computer program is configured to execute, when running, a method for adjusting a dental model described above.

One embodiment of the present disclosure further provides a processor, configured to run a program. The computer program is configured to execute, when running, a method for adjusting a dental model described above.

In the embodiments of the present disclosure, a manner of adjusting the initial edge line of the dental model is adopted. After the initial edge line of the dental model to be adjusted is acquired, the at least part of the initial edge line is adjusted to obtain the adjusted edge line.

In the above process, by adjusting the initial edge line of the dental model, the adjusted edge line is more accurate, which facilitates design of subsequent dental restoration. Moreover, in the above process, the operator can restore the dental model without strong software operation ability, which lowers the requirements for the skill of the operator. In addition, in the above process, the initial edge line is adjusted automatically, and there is no need to redraw the initial edge line, which saves the time cost and improves the correction efficiency of the dental model.

As can be seen, the solutions provided by this application achieve the purpose of adjusting the dental model, so that the accuracy of trimming the edge line of the dental model is improved, thereby solving the problem of inaccurate edge line after trimming the dental model in the prior art.

### Brief Description of the Drawings

The accompanying drawings described herein are used to provide a further understanding of the present disclosure, and form part of the present disclosure. Exemplary embodiments of the present disclosure and descriptions thereof are used to explain the present disclosure, and do not constitute any inappropriate limitation to the present disclosure. In the accompanying drawings:
FIG. 1 is a flowchart of a method for adjusting a dental model according to an embodiment of the present disclosure;
FIG. 2 is a schematic diagram of an optional display interface according to an embodiment of the present disclosure;
FIG. 3 is a schematic diagram of an optional display interface according to an embodiment of the present disclosure;
FIG. 4 is a schematic diagram of an optional depth map according to an embodiment of the present disclosure;
FIG. 5 is a schematic diagram of an optional display interface according to an embodiment of the present disclosure;
FIG. 6 is a schematic diagram of an optional occlusion area according to an embodiment of the present disclosure;
FIG. 7 is a schematic diagram of optional undercut filling according to an embodiment of the present disclosure;
FIG. 8 is a schematic diagram of an optional dental model according to an embodiment of the present disclosure;
FIG. 9 is a schematic diagram of an optional dental model according to an embodiment of the present disclosure;
FIG. 10 is a schematic diagram of an optional dental model according to an embodiment of the present disclosure;
FIG. 11 is a schematic diagram of an optional dental model according to an embodiment of the present disclosure; and
FIG. 12 is a schematic diagram of an device for adjusting a dental model according to an embodiment of the present disclosure.

### Detailed Description of the Invention

In order to make those skilled in the art better understand the solutions of the present disclosure, the technical solutions in the embodiments of the present disclosure will be clearly and completely described below in conjunction with the accompanying drawings in the embodiments of the present disclosure. It is apparent that the described embodiments are merely some embodiments, but not all embodiments of the present disclosure. All other embodiments obtained by those of ordinary skill in the art based on the embodiments of the present disclosure without creative work shall fall into the protection scope of the present disclosure.

It should be noted that the terms "first", "second" and the like in the specification, claims and accompanying drawings of the present disclosure are used to distinguish similar objects, and are not necessarily used to describe a particular order or sequence. It should be understood that the data used as such may be interchanged where appropriate, so that the embodiments of the present disclosure described herein can be implemented in a sequence other than those illustrated or described herein. In addition, the terms "including" and "having" and any variants thereof are intended to cover a non-exclusive inclusion. For example, a process, method, system, product, or device that includes a series of steps or units is not necessarily limited to those steps or units that are clearly listed, but may include other steps or units that are not clearly listed or inherent to those processes, methods, products or devices.

### Embodiment 1

According to this embodiment of the present disclosure, provided is an embodiment of a method for adjusting a dental model. It should be noted that steps shown in the flowchart of the accompanying drawing may be executed in a computer system such as a set of computer-executable instructions. In addition, although a logical order is shown in the flowchart, in some cases, the steps shown or described may be executed in a different order than here.

Additionally, it should also be noted that a terminal device may be used as the execution subject of the method for adjusting a dental model. The terminal device at least includes an input unit and an output unit. Alternatively, the output unit at least includes a display.

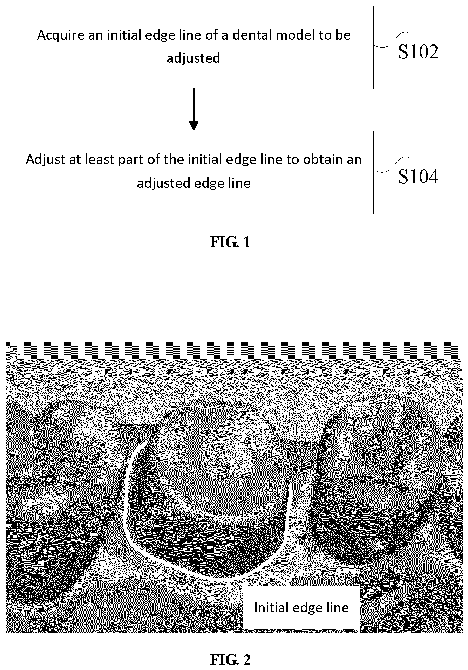

In addition, FIG. 1 a flowchart of a method for adjusting a dental model according to an embodiment of the present disclosure. As shown in FIG. 1, the method includes the following steps:
Step S102: Acquiring an initial edge line of a dental model to be adjusted.

In step S102, the initial edge line of the dental model to be adjusted may be determined by the user. For example, the user sets the initial edge line of the dental model to be adjusted by means of the input unit of the terminal device. For example, in the schematic diagram of the display interface of the terminal device shown in FIG. 2, the white line is the initial edge line, that is, the white line is the initial edge line input by the user.

It should be noted that the user needs to input not only the initial edge line to the terminal device, but also the dental model to be adjusted and a path of insertion. The path of insertion is the direction of the denture when it is placed into the mouth.

Step S104: Adjusting at least part of the initial edge line to obtain an adjusted edge line.

Alternatively, in step S104, the terminal device first controls the initial edge line to sink along the direction of the path of insertion. After the initial edge line sinks to a specified position, the initial edge line is deformed to obtain the adjusted edge line.

It should be noted that in practical applications, the user may choose to adjust the whole of the initial edge line or only part of the edge line according to needs.

Additionally, it should also be noted that after the initial edge line sinks to the specified position, the initial edge line is deformed to keep the smoothness of the edge line.

Based on the solution defined in step S102 to step S104, it can be known that in this embodiment of the present disclosure, a manner of adjusting the initial edge line of the dental model is adopted. After the initial edge line of the dental model to be adjusted is acquired, the at least part of the initial edge line is adjusted to obtain the adjusted edge line.

It is easily noted that in the above process, the initial edge line of the dental model is adjusted, so that the adjusted edge line is more accurate, which facilitates design of subsequent dental restoration. Moreover, in the above process, the operator can restore the dental model without strong software operation ability, which lowers the requirements for the skill of the operator. In addition, in the above process, the initial edge line is adjusted automatically, and there is no need to redraw the initial edge line, which saves the time cost and improves the correction efficiency of the dental model.

As can be seen, the solutions provided by this application achieve the purpose of adjusting the dental model, so that the accuracy of trimming the edge line of the dental model is improved, thereby solving the problem of inaccurate edge line after trimming the dental model in the prior art.

In an optional embodiment, before the dental model is adjusted, the terminal device needs to acquire the initial edge line of the dental model to be adjusted. Specifically, the terminal device projects the dental model to be adjusted to obtain a target area corresponding to the dental model to be adjusted, and then determines the initial edge line according to the target area. The target area at least includes a deformation area.

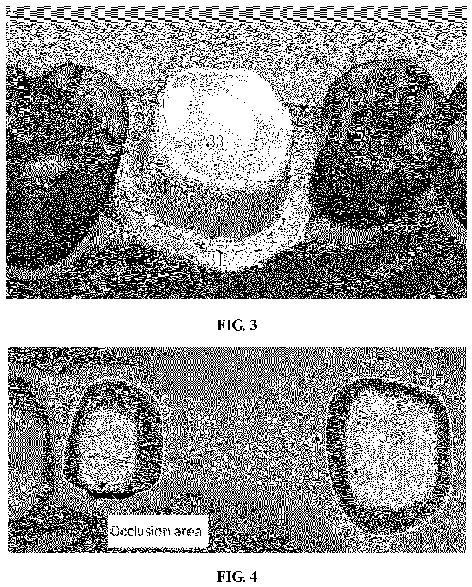

Alternatively, the terminal device projects the dental model to be adjusted to obtain the target area. For example, in the schematic diagram of the display interface shown in FIG. 3, the target area is the area contained in curve 32. The area contained in curve 31 is the projection area. The light-colored area contained in the curve 32 is the deformation area. The dark-colored area contained in the curve 32 is the constraint area. The dashed line 33 is the direction of the path of insertion. Alternatively, curve 30 is the initial edge line set by the user.

Further, after the initial edge line of the dental model to be adjusted is determined, the terminal device detects whether the dental model to be adjusted has a defect. Specifically, the terminal device detects whether the dental model to be adjusted has a defect. The terminal device adjusts, in a case that the dental model to be adjusted does not have the defect, the dental model to be adjusted based on the initial edge line to obtain the adjusted edge line; and perform, in a case that the dental model to be adjusted has the defect, a filling operation on the dental model to be adjusted based on the defect to obtain a filled dental model to be adjusted, adjust the filled dental model to be adjusted based on the initial edge line to obtain a target model, and adjust the at least part of the initial edge line based on the target model to obtain the adjusted edge line.

It should be noted that the above defect may be, but not limited to, an undercut of the dental model. If the dental model to be adjusted has the undercut, the position of the edge line of the dental model to be adjusted may be affected. Therefore, before the edge line of the dental model to be adjusted is adjusted, it is required to detect whether the dental model to be adjusted has the undercut. If the dental model to be adjusted does not have the undercut, the edge line of the dental model to be adjusted can be adjusted directly. Otherwise, after undercut filling is performed on the dental model to be adjusted to obtain the target model, the edge line is adjusted based on the target model.

In an optional embodiment, the terminal device may detect whether the dental model to be adjusted has the defect according to a depth map of the dental model to be adjusted. Specifically, the terminal device first acquires a target area of the dental model to be adjusted and a depth map corresponding the dental model to be adjusted, then determines, according to the depth map, whether there is an occlusion area in the target area of the dental model to be adjusted, and finally, determines, in a case that there is the occlusion area in the target area of the dental model to be adjusted, that the dental model to be adjusted has the defect.

Alternatively, the terminal device first rotates the dental model to be adjusted such that the path of insertion of the dental model to be adjusted is in the Z-axis direction; and then, establishes the depth map of the dental model to be adjusted. For example, in the depth map shown in FIG. 4, the black area is the occlusion area. Thereby, it can be determined that the dental model on the left in FIG. 4 has the defect, and it is required to perform undercut filling on the dental model on the left.

It should be noted that the depth map is depth information of the dental model to be adjusted in the direction of the path of insertion, i.e., the depth position of the dental model to be adjusted projected in the direction of the path of insertion. The depth map can be used as an instruction for subsequently determining the occlusion area and the position of the occlusion point to be deformed.

Alternatively, the terminal device first slices the dental model to be adjusted to obtain the occlusion area, then acquires occlusion points contained in the occlusion area according to the depth map and the layer where the dental model to be adjusted is located, and records positions to be moved corresponding to the occlusion points. The positions to be moved corresponding to the occlusion points are nearest points on the contour line of the depth map.

In an optional embodiment, in a case that the dental model to be adjusted has the defect, it is required to perform an undercut filling operation on the dental model to be adjusted. Specifically, the terminal device first performs a culling operation on noise information in a defect area to obtain a first defect area, then mixes isolated occlusion points in the first defect area to obtain a second defect area, and finally, performs a filling operation on the second defect area to obtain the filled dental model to be adjusted. The defect area includes a plurality of occlusion points.

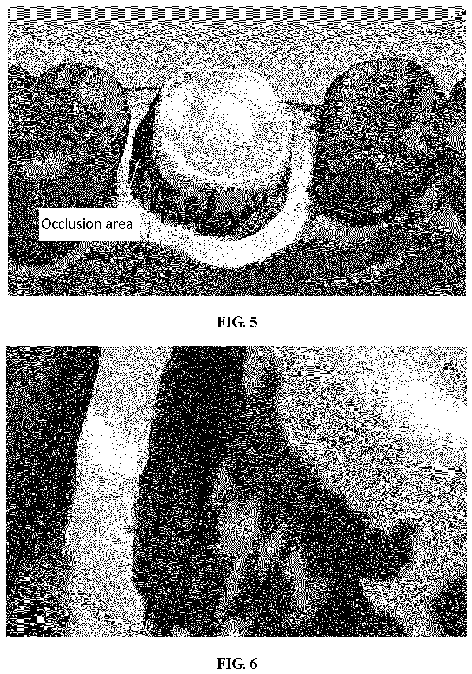

For example, an optimization operation is performed on the occlusion area in FIG. 5. The terminal device first removes the minimal occlusion area caused by noise or accuracy, mixes isolated points in the occlusion area to obtain the second defect area, and then performs the filling operation on the second defect area. For example, FIG. 6 is a schematic diagram of the occlusion area. In FIG. 6, the lines in the occlusion area are the corresponding points when performing the filling operation on the dental model to be adjusted. The terminal device may deform the dental model to be adjusted in an iterative manner, thereby completing the filling of the occlusion area.

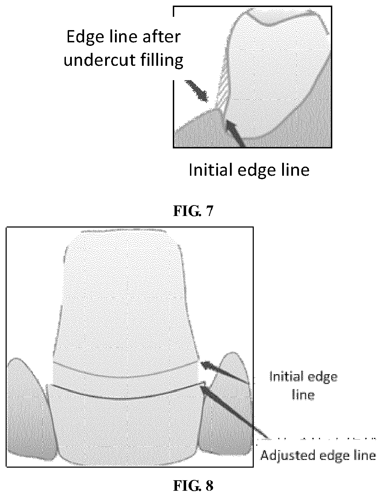

It should be noted that since the undercut filling operation may cover part of the original edge line in the dental model to be adjusted, after the undercut filling is performed on the dental model to be adjusted, it is required to determine whether to adjust the edge line of the dental model to be adjusted to adapt to the undercut. The line where the undercut intersects the gum becomes the edge line after the undercut filling, as shown in FIG. 7.

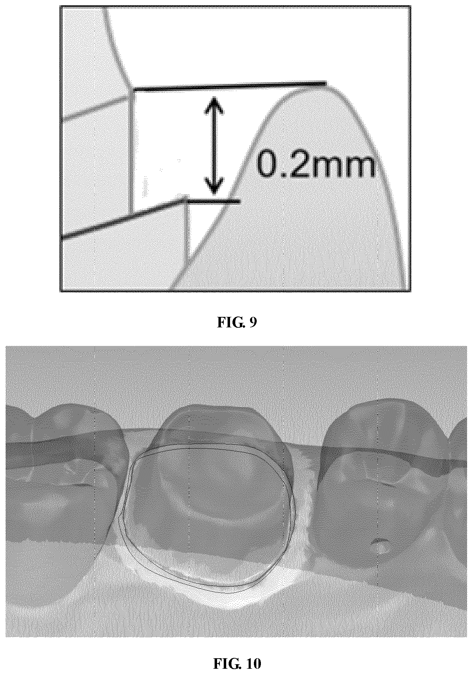

Additionally, it should also be noted that in the process of making the dental model, it is required to perform edge carving on the dental model. The edge carving is based on the edge line that is recognizable by the naked eye. However, in practical applications, the edge line may not be accurate. In addition, in the process of taking the dental model, the gum may press the edge line, so that the edge line cannot be fully exposed. Therefore, it is required to deform the edge line. The deformation may be vertical sinking based on the edge line, i.e., the edge line sinks downward according to the set direction of the path of insertion, and the upper part of the model does not need to be deformed. The sinking distance is specified by the user. For example, in the dental model shown in FIG. 8, initial edge line is not accurate, and it is required to perform a sinking operation on the initial edge line such that the initial edge line sinks to the position of the adjusted edge line. FIG. 9 shows that the sinking distance of the initial edge line is 0.2 mm.

In an optional embodiment, in a case that the dental model to be adjusted does not have the defect and an accuracy of a whole of the initial edge line is lower than a preset accuracy, that a movement mode of the at least part of the initial edge line is a first movement mode. The first movement mode is used for controlling the whole of the initial edge line to a preset position.

It should be noted that in the first movement mode, the initial edge line moves downward as a whole, i.e., the whole initial edge line moves downward according to the set sinking distance. The initial edge line is vertically pulled down to form a new edge line (i.e., adjusted edge line), and the upper part of the dental model to be adjusted does not need to be deformed.

In an optional embodiment, in a case that the dental model to be adjusted does not have the defect and an accuracy of the part of the initial edge line is lower than a preset accuracy, it is determined that a movement mode of the at least part of the initial edge line is a second movement mode. In the second movement mode, the terminal device first determines a start position and an end position of the at least part of the initial edge line based on the target model, then determines a first edge line according to the start position and the end position, and finally controls the first edge line to move to a preset position to obtain the adjusted edge line. An accuracy of the first edge line is lower than the preset accuracy.

It should be noted that in the second movement mode, control points are selected, and the first control point (i.e., start position) and the last control point (i.e., end position) in the selected control points are set as fixed points; and the edge line in the middle (i.e., the first edge line) sinks as a whole according to the set sinking distance so that the sinking part forms a new edge line (i.e., adjusted edge line), and the upper part of the dental model to be adjusted does not need to be deformed.

In an optional embodiment, in a case that the dental model to be adjusted has the defect and an accuracy of the part of the initial edge line is lower than a preset accuracy, it is determined that a movement mode of the at least part of the initial edge line is a third movement mode. In the third movement mode, the terminal device first determines a start position and an end position of the at least part of the initial edge line, and determines a second edge line according to the start position and the end position; then determines critical control points from a plurality of control points contained in the second edge line, and determines a preset position corresponding to each of the critical control points; and finally, controls each of the critical control points to move to the preset position corresponding to each of the critical control points to obtain the adjusted edge line. In terms of non-critical control points, target positions corresponding to non-critical control points contained in the second edge line are first determined; and then, it is determined that the non-critical control points are moved to the target positions to obtain the adjusted edge line. An accuracy of the second edge line is lower than the preset accuracy.

It should be noted that the third movement mode is usually applied to a scene where the newly generated edge line after the undercut filling sinks. In the third movement mode, part of the edge line remains unchanged, and the sinking distance of each control point in the remaining part of the edge line (i.e., second edge line) is different. In this mode, the user may specify the critical control points and respectively set the sinking distance corresponding to each critical control point, and then control the critical control points to sink according to the respective sinking distance, and the rest of the non-critical control points in the second edge line adaptively descend to the target positions, so that the position coordinates of the critical control points after sinking and the position coordinates of the non-critical control points after sinking are on a smooth curve.

In some embodiments, the terminal device acquires a movement value and the movement mode of the dental model to be adjusted, and controls the at least part of the initial edge line to move from the current position to the preset position according to the movement mode to obtain the adjusted edge line. The movement value is a distance between the current position and the preset position, and the preset position is in the deformation area.

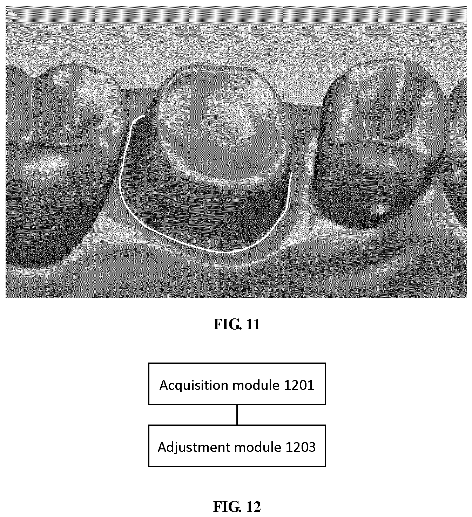

It should be noted that the movement modes include the first movement mode, the second movement mode and the third movement mode. The user may determine the movement mode to be used from the three movement modes according to actual needs. Alternatively, in the schematic diagram shown in FIG. 10, the area contained in the two solid lines is the position where the initial edge line of the dental model to be adjusted will sink. FIG. 11 shows a schematic diagram of the adjusted edge line in the dental model to be adjusted.

As can be seen from the above contents, in this application, by pre-marking the edge line and the path of insertion and presetting the value of sinking of the whole or part of the edge line, the edge line automatically sinks according to the direction of the path of insertion, and at the same time, the sunk edge line is smoothed to ensure the smoothness of the edge line.

By means of the above method, the initial edge line and the path of insertion may be determined by doctors or experienced technicians, and the sinking distance of the initial edge line may be set accurately to avoid errors caused by determining whether the edge line has been adjusted to into position according to the hand feeling in the prior art. In addition, in this application, the deformation intensity and range of the initial edge line can be adjusted flexibly without redrawing the edge line, which improves the flexibility of correcting the dental model.

### Embodiment 2

According to this embodiment of the present disclosure, further provided is an embodiment of an device for adjusting a dental model. FIG. 12 is a schematic diagram of an device for adjusting a dental model according to an embodiment of the present disclosure. As shown in FIG. 12, the device includes: an acquisition module 1201 and an adjustment module 1203.

The acquisition module 1201 is configured to acquire an initial edge line of a dental model to be adjusted. The adjustment module 1203 is configured to adjust at least part of the initial edge line to obtain an adjusted edge line.

It should be noted that the acquisition module 1201 and the adjustment module 1203 correspond to step S 102 to step S104 in the above embodiment, and the examples and application scenarios realized by the two modules are the same as those of the corresponding steps, but are not limited to the contents disclosed in Embodiment 1.

Alternatively, the acquisition module includes: a projection module and a first determination module. The projection module is configured to project the dental model to be adjusted to obtain a target area corresponding to the dental model to be adjusted. The target area at least includes a deformation area. The first determination module is configured to determine the initial edge line according to the target area.

Alternatively, the adjustment module includes: a detection module, a first adjustment module and a second adjustment module. The detection module is configured to detect whether the dental model to be adjusted has a defect. The first adjustment module is configured to adjust, in a case that the dental model to be adjusted does not have the defect, the dental model to be adjusted based on the initial edge line to obtain the adjusted edge line. The second adjustment module is configured to perform, in a case that the dental model to be adjusted has the defect, a filling operation on the dental model to be adjusted based on the defect to obtain a filled dental model to be adjusted, adjust the filled dental model to be adjusted based on the initial edge line to obtain a target model, and adjust the at least part of the initial edge line based on the target model to obtain the adjusted edge line.

Alternatively, the detection module includes: a first acquisition module, a second determination module and a third determination module. The first acquisition module is configured to acquire a target area of the dental model to be adjusted and a depth map corresponding the dental model to be adjusted. The second determination module is configured to determine, according to the depth map, whether there is an occlusion area in the target area of the dental model to be adjusted. The third determination module is configured to determine, in a case that there is the occlusion area in the target area of the dental model to be adjusted, that the dental model to be adjusted has the defect.

Alternatively, the second adjustment module includes: a culling module, a processing module and a filling module. The culling module is configured to perform a culling operation on noise information in a defect area to obtain a first defect area. The defect area includes a plurality of occlusion points. The processing module is configured to mix isolated occlusion points in the first defect area to obtain a second defect area. The filling module is configured to perform a filling operation on the second defect area to obtain the filled dental model to be adjusted.

Alternatively, the device for adjusting a dental model further includes: a fourth determination module. The fourth determination module is configured to determine, in a case that the dental model to be adjusted does not have the defect and an accuracy of a whole of the initial edge line is lower than a preset accuracy, that a movement mode of the at least part of the initial edge line is a first movement mode. The first movement mode is used for controlling the whole of the initial edge line to a preset position.

Alternatively, the device for adjusting a dental model further includes: a fifth determination module. The fifth determination module is configured to determine, in a case that the dental model to be adjusted does not have the defect and an accuracy of the part of the initial edge line is lower than a preset accuracy, that a movement mode of the at least part of the initial edge line is a second movement mode.

Alternatively, the adjustment module includes: a sixth determination module, a seventh determination module and a first control module. The sixth determination module is configured to determine, based on the target model, a start position and an end position of the at least part of the initial edge line. The seventh determination module is configured to determine a first edge line according to the start position and the end position. An accuracy of the first edge line is lower than the preset accuracy. The first control module is configured to control the first edge line to move to a preset position to obtain the adjusted edge line.

Alternatively, the device for adjusting a dental model further includes: an eighth determination module. The eighth determination module is configured to determine, in a case that the dental model to be adjusted has the defect and an accuracy of the part of the initial edge line is lower than a preset accuracy, that a movement mode of the at least part of the initial edge line is a third movement mode.

Alternatively, the second adjustment module includes: a ninth determination module, a tenth determination module, an eleventh determination module, a twelfth determination module and a second control module. The ninth determination module is configured to determine, based on the target model, a start position and an end position of the at least part of the initial edge line. The tenth determination module is configured to determine a second edge line according to the start position and the end position. An accuracy of the second edge line is lower than the preset accuracy. The eleventh determination module is configured to determine critical control points from a plurality of control points contained in the second edge line. The twelfth determination module is configured to determine a preset position corresponding to each of the critical control points. The second control module is configured to control each of the critical control points to move to the preset position corresponding to each of the critical control points to obtain the adjusted edge line.

Alternatively, the device for adjusting a dental model further includes: a thirteenth determination module and a fourteenth determination module. The thirteenth determination module is configured to determine target positions corresponding to non-critical control points contained in the second edge line. The fourteenth determination module is configured to determine that the non-critical control points are moved to the target positions to obtain the adjusted edge line.

### Embodiment 3

According to another aspect, an embodiment of the present disclosure further provides a non-volatile storage medium, storing a computer program therein. The computer program is configured to execute, when running, the method for adjusting a dental model in Embodiment 1.

### Embodiment 4

According to another aspect, an embodiment of the present disclosure further provides a processor, configured to run a program. The program is configured to execute, when running, the method for adjusting a dental model in Embodiment 1.

The sequence numbers of the embodiments of the present disclosure are merely for description purposes but do not imply the preference among the embodiments.

In the above embodiments of the present disclosure, the description for each embodiment has its own focus. For parts that are not described in detail in a certain embodiment, reference may be made to related descriptions of other embodiments.

In the several embodiments provided in this application, it should be understood that the disclosed technical contents can be implemented in other ways. The embodiment of the device described above is only schematic. For example, the division of units may be only a division of logical functions. In an actual implementation, there may be other division manners, for example, a plurality of units or components may be combined or may be integrated into another system, or some features may be omitted or not executed. In addition, the displayed or discussed mutual coupling or direct coupling or communication connection may be indirect coupling or communication connection through some interfaces, units or modules, and may be in electrical or other forms.

The units described as separate components may or may not be physically separated, and the components displayed as units may or may not be physical units, that is, they may be located in one place, or may be distributed in a plurality of units. Part or all of the units may be selected according to actual needs to achieve the purposes of the solution of this embodiment.

In addition, the function units in each embodiment of the present disclosure may be integrated into one processing unit, or each unit may exist alone physically, or two or more units may be integrated into one unit. The above integrated unit may be implemented in the form of either hardware or software functional units.

If the integrated unit is achieved in the form of a software function module and sold or used as an independent product, the integrated unit may be stored in a computer-readable storage medium. Based on such an understanding, the technical solution of the present disclosure essentially or the part that contributes to the prior art or all or part of the technical solution can be embodied in the form of a software product, and the computer software product is stored in a storage medium and includes several instructions configured to enable a computer device (which may be a personal computer, a server, a network device or the like) to execute all or part of the steps of the methods of the embodiments of the present disclosure. The foregoing storage medium includes: a USB flash disk, a read-only memory (ROM), a random access memory (RAM), a mobile hard disk, a magnetic disk, an optical disk, or various other media that can store program codes.

The above description is only preferred implementations of the present disclosure. It should be noted that those of ordinary skill in the art may make several improvements and modifications without departing from the principles of the present disclosure, and such improvements and modifications should also be regarded as the protection scope of the present disclosure.

### Industrial applicability

The solutions provided by the embodiments of the present disclosure can be applied to the aspect of oral diagnosis, treatment and restoration. The initial edge line of the dental model is adjusted automatically, and there is no need to redraw the initial edge line, which improves the correction efficiency of the dental model. Thereby, the adjusted edge line is more accurate, which facilitates design of subsequent dental restoration.

## Claims

1. A method for adjusting a dental model, comprising:
acquiring an initial edge line of the dental model to be adjusted; and
adjusting at least part of the initial edge line to obtain an adjusted edge line.

2. The method as claimed in claim 1, wherein the acquiring the initial edge line of the dental model to be adjusted comprises:
projecting the dental model to be adjusted to obtain a target area corresponding to the dental model to be adjusted, the target area at least comprising a deformation area; and
determining the initial edge line according to the target area.

3. The method as claimed in claim 1, wherein the adjusting at least part of the initial edge line to obtain the adjusted edge line comprises:
detecting whether the dental model to be adjusted has a defect;
adjusting, in a case that the dental model to be adjusted does not have the defect, the dental model to be adjusted based on the initial edge line to obtain the adjusted edge line; and
performing, in a case that the dental model to be adjusted has the defect, a filling operation on the dental model to be adjusted based on the defect to obtain a filled dental model to be adjusted, adjusting the filled dental model to be adjusted based on the initial edge line to obtain a target model, and adjusting the at least part of the initial edge line based on the target model to obtain the adjusted edge line.

4. The method as claimed in claim 3, wherein the detecting whether the dental model to be adjusted has the defect comprises:
acquiring a target area of the dental model to be adjusted and a depth map corresponding the dental model to be adjusted;
determining, according to the depth map, whether there is an occlusion area in the target area of the dental model to be adjusted; and
determining, in a case that there is the occlusion area in the target area of the dental model to be adjusted, that the dental model to be adjusted has the defect.

5. The method as claimed in claim 3, wherein the performing, in the case that the dental model to be adjusted has the defect, the filling operation on the dental model to be adjusted based on the defect to obtain the filled dental model to be adjusted comprises:
performing a culling operation on noise information in a defect area to obtain a first defect area, the defect area comprising a plurality of occlusion points;
mixing isolated occlusion points in the first defect area to obtain a second defect area; and
performing a filling operation on the second defect area to obtain the filled dental model to be adjusted.

6. The method as claimed in claim 3, wherein the method further comprises:
determining, in a case that the dental model to be adjusted does not have the defect and an accuracy of a whole of the initial edge line is lower than a preset accuracy, that a movement mode of the at least part of the initial edge line is a first movement mode, the first movement mode being used for controlling the whole of the initial edge line to a preset position.

7. The method as claimed in claim 3, wherein the method further comprises:
determining, in a case that the dental model to be adjusted does not have the defect and an accuracy of the part of the initial edge line is lower than a preset accuracy, that a movement mode of the at least part of the initial edge line is a second movement mode.

8. The method as claimed in claim 7, wherein the adjusting at least part of the initial edge line to obtain the adjusted edge line comprises:
determining, based on the target model, a start position and an end position of the at least part of the initial edge line;
determining a first edge line according to the start position and the end position, an accuracy of the first edge line being lower than the preset accuracy; and
controlling the first edge line to move to a preset position to obtain the adjusted edge line.

9. The method as claimed in claim 3, wherein the method further comprises
determining, in a case that the dental model to be adjusted has the defect and an accuracy of the part of the initial edge line is lower than a preset accuracy, that a movement mode of the at least part of the initial edge line is a third movement mode.

10. The method as claimed in claim 9, wherein the adjusting the at least part of the initial edge line based on the target model to obtain the adjusted edge line comprises:
determining, based on the target model, a start position and an end position of the at least part of the initial edge line;
determining a second edge line according to the start position and the end position, an accuracy of the second edge line being lower than the preset accuracy;
determining critical control points from a plurality of control points contained in the second edge line;
determining a preset position corresponding to each of the critical control points; and
controlling each of the critical control points to move to the preset position corresponding to each of the critical control points to obtain the adjusted edge line.

11. The method as claimed in claim 10, wherein the method further comprises:
determining target positions corresponding to non-critical control points contained in the second edge line; and
determining that the non-critical control points are moved to the target positions to obtain the adjusted edge line.

12. A device for adjusting a dental model, comprising:
an acquisition module, configured to acquire an initial edge line of a dental model to be adjusted; and
an adjustment module, configured to adjust at least part of the initial edge line to obtain an adjusted edge line.

13. A non-volatile storage medium, storing a computer program therein, wherein the computer program is configured to execute, when running, a method for adjusting a dental model as claimed in any one of claims 1 to 11.

14. A processor, configured to run a program, wherein the program is configured to execute, when running, a method for adjusting a dental model as claimed in any one of claims 1 to 11.
